# FASCICULE DE BREVET EUROPEEN

(11) **EP 3 446 099 B1**
(45) Date de publication et mention de la délivrance du brevet: **08.01.2020**
(21) Numéro de dépôt: 17716555.2
(22) Date de dépôt: 14.04.2017
(51) Int. Cl.: G01N 21/35, A61K 31/702, C07H 3/06, G01N 33/66

(54) **PROCÉDÉ DE SÉQUENÇAGE D'OLIGOSACCHARIDES**
VERFAHREN ZUR SEQUENZIERUNG VON OLIGOSACCHARIDEN
METHOD FOR SEQUENCING OLIGOSACCHARIDES

(30) Priorité: 18.04.2016 FR 1653425
(43) Date de publication de la demande: 27.02.2019
(73) Titulaire: Centre National de la Recherche Scientifique, 75794 Paris Cedex 16 (FR); Université Claude Bernard Lyon 1, 69100 Villeurbanne (FR)
(72) Inventeur: COMPAGNON, Isabelle, 69005 Lyon (FR); SCHINDLER, Baptiste, 69100 Villeurbanne (FR)
(74) Mandataire: Be IP
(86) Numéro de dépôt international: PCT/EP2017/059050
(87) Numéro de publication internationale: WO 2017/182404

(56) Documents cités:
- PIKULSKI M ET AL: "Sequencing and Characterization of Oligosaccharides Using Infrared Multiphoton Dissociation and Boronic Acid Derivatization in a Quadrupole Ion Trap", JOURNAL OF THE AMERICAN SOCIETY FOR MASS SPECTROMETRY, ELSEVIER SCIENCE INC, US, vol. 18, no. 12, 14 septembre 2007 (2007-09-14), pages 2094-2106, XP026268075, ISSN: 1044-0305, DOI: 10.1016/J.JASMS.2007.09.005 [extrait le 2007-12-01]
- SARAH E. STEFAN ET AL: "Differentiation of Closely Related Isomers: Application of Data Mining Techniques in Conjunction with Variable Wavelength Infrared Multiple Photon Dissociation Mass Spectrometry for Identification of Glucose-Containing Disaccharide Ions", ANALYTICAL CHEMISTRY, vol. 83, no. 22, 29 septembre 2011 (2011-09-29), pages 8468-8476, XP055326167, US ISSN: 0003-2700, DOI: 10.1021/ac2017103 cité dans la demande
- BAPTISTE SCHINDLER ET AL: "Distinguishing isobaric phosphated and sulfated carbohydrates by coupling of mass spectrometry with gas phase vibrational spectroscopy", PHYSICAL CHEMISTRY CHEMICAL PHYSICS., vol. 16, no. 40, 28 août 2014 (2014-08-28) , pages 22131-22138, XP055325850, GB ISSN: 1463-9076, DOI: 10.1039/C4CP02898H cité dans la demande
- SHUAI WU ET AL: "Employment of Tandem Mass Spectrometry for the Accurate and Specific Identification of Oligosaccharide Structures", ANALYTICAL CHEMISTRY, vol. 84, no. 17, 6 août 2012 (2012-08-06), pages 7456-7462, XP055327565, US ISSN: 0003-2700, DOI: 10.1021/ac301398h
- AMANDA L. PATRICK ET AL: "Insights into the fragmentation pathways of gas-phase protonated sulfoserine", INTERNATIONAL JOURNAL OF MASS SPECTROMETRY., vol. 379, 23 décembre 2014 (2014-12-23), pages 26-32, XP055325881, NL ISSN: 1387-3806, DOI: 10.1016/j.ijms.2014.12.001
- MELLISA LY ET AL: "The proteoglycan bikunin has a defined sequence", NATURE CHEMICAL BIOLOGY, vol. 7, no. 11, 9 octobre 2011 (2011-10-09), pages 827-833, XP055326173, GB ISSN: 1552-4450, DOI: 10.1038/nchembio.673 cité dans la demande

## Description

### DOMAINE DE L'INVENTION

La présente invention concerne un procédé de séquençage d'oligosaccharides, qui permet d'identifier la séquence primaire d'oligosaccharides de structure inconnue, incluant sa composition en monosaccharides, la position (régiochimie) et la configuration (stéréochimie) des liaisons glycosidiques, la nature et la position des modifications fonctionnelles, et sa structure branchée, dont en particulier l'identification de l'extrémité réductrice.

### ETAT DE LA TECHNIQUE

Le séquençage à l'échelle industrielle des protéines et du génome a eu un impact économique et scientifique considérable depuis les années 90 et a révolutionné la biologie moderne. Cependant, il n'existe aucune méthode commerciale permettant le séquençage systématique des saccharides, du fait d'une complexité structurelle supérieure aux autres classes de biopolymères. Le manque d'outils d'analyse adaptés à la structure moléculaire spécifique des saccharides constitue un frein au développement des glycosciences.

Deux approches peuvent être envisagées pour résoudre la structure de biomolécules: soit une approche globale de la structure (par exemple par Résonnance Magnétique Nucléaire - RMN), soit une approche de type «séquençage» qui consiste en la réduction du polymère en sous-unités et en l'analyse de la structure de ces sous-unités. Idéalement, le détail de la structure de la molécule d'intérêt est préservé dans ses sous-unités et peut être retrouvé par l'analyse de celles-ci. Ce prérequis est vérifié par exemple dans le cas du séquençage de protéines par spectrométrie de masse.

Dans le cas des sucres, plusieurs méthodes permettent de réduire un saccharide en sous-unités, dont la digestion enzymatique, l'hydrolyse chimique, et diverses méthodes de fragmentation par spectrométrie de masse.

La principale technologie décrite en rapport avec le séquençage d'oligosaccharides est transposée du séquençage de protéines et repose sur la fragmentation et l'analyse des fragments par spectrométrie de masse (Science 1999, 15, 537-542; Nature Chem. Biol. 2011, 7, 827-833). Ce type d'analyse ne permet pas de déterminer toutes les propriétés structurales pertinentes des fragments, et ses limitations sont notoires et largement référencées (Omics 2010, 14, 401-418).

Plus récemment, plusieurs groupes ont proposé une combinaison de la mobilité ionique avec la spectrométrie de masse (Li, H. & al., Rapid Comm. Mass Spectrom. 2013, 27, 2699-2709; Both, P. & al., Nat. Chem. 2013, 6, 65-74; Gaye, M. M. & al., Analyst, 2015, 140, 6922; Hofmann, J. & al., Nature, 2015, 526, 241). Cette technologie n'a pas permis d'établir une relation directe entre la structure de la molécule d'intérêt et la structure de ses fragments.

Plus récemment, Nagy & al. (Anal. Chem. 2015, 87, 677-685; J. Amer. Soc. Mass Spectrom. 2015, 26, 677-685 ; Anal. Chem. 2016, 88, 2335-2344) décrivent l'analyse de monosaccharides par une méthode de complexation avec un métal divalent et un référent chiral, comme un acide aminé (L-aspartique ou L-serine) puis analyse de la masse des complexes formés par spectrométrie de masse. L'analyse *de novo* envisagée par les auteurs nécessite une hydrolyse préalable des oligosaccharides et une préparation des échantillons qui reste à développer.

Ces publications de recherche fondamentale concernent l'évaluation de techniques orthogonales à la spectrométrie de masse, mais ne permettent pas la résolution *de novo* de séquences d'oligosaccharides de structures inconnues. En particulier, elles ne permettent pas d'obtenir les informations nécessaires au séquençage complet des oligosaccharides, comprenant sa composition en monosaccharides, la position (régiochimie) et la configuration (stéréochimie) des liaisons glycosidiques, la nature et la position des modifications fonctionnelles, et sa structure branchée, dont en particulier l'identification de l'extrémité réductrice.

Le procédé selon l'invention permet de résoudre ce problème.

L'homme du métier connaît différentes méthodes d'analyse de mono ou disaccharides par spectroscopie vibrationnelle, notamment des méthodes où les échantillons purs de mono ou disaccharides sont préparés au moyen d'un appareil de spectrométrie de masse qui est employé à des fins de préparation des échantillons en phase gazeuse pour l'analyse par spectroscopie vibrationnelle, et non pas pour leur fragmentation (Stefan, S. & al., Anal. Chem. 2011, 83, 8468-8476 ; Schindler, B. & al., Phys. Chem. Chem. Phys. 2014, 16, 22131-22138).

PIKULSKI M ET AL ("Sequencing and Characterization of Oligosaccharides Using Infrared Multiphoton Dissociation and Boronic Acid Derivatization in a Quadrupole Ion Trap", JOURNAL OF THE AMERICAN SOCIETY FOR MASS SPECTROMETRY, ELSEVIER SCIENCE INC, US, vol. 18, no. 12, 14 septembre 2007 (2007-09-14), pages 2094-2106, ISSN: 1044-0305, DOI: 10.1016/ J.JASMS.2007.09.005) décrivent un procédé de séquençage d'oligosaccharides comprenant les étapes de fragmentation des oligosaccharides par IRMPD, séparation de chaque fragment obtenus précédemment par spectrométrie de masse, et définition de la séquence de l'oligosaccharide par combinaison des structures identifiées.

### EXPOSE DE L'INVENTION

La présente invention est définie dans la revendication 1 et concerne un procédé de séquençage d'oligosaccharides caractérisé en ce qu'il comprend les étapes de
i. fragmentation des oligosaccharides en disaccharides et monosaccharides en préservant la structure moléculaire des constituants telle que présente dans l'oligosaccharide à séquencer
ii. séparation de chaque disaccharide et monosaccharide obtenus précédemment par spectrométrie de masse,
iii. analyse par spectroscopie vibrationnelle IR (infrarouge) de chaque disaccharide et monosaccharide séparés précédemment,
iv. identification de la structure de chaque disaccharides et monosaccharides par comparaison des spectres IR obtenus à un ensemble de spectres IR de disaccharides et monosaccharides de référence, et
v. définition de la séquence de l'oligosaccharide par combinaison des structures identifiées pour chaque disaccharide et monosaccharide.

L'invention concerne aussi un dispositif selon la revendication 6 pour la mise en œuvre du procédé, qui est défini dans la revendication 1.

### DESCRIPTION DETAILLEE DE L'INVENTION

La présente invention concerne un procédé de séquençage d'oligosaccharides qui permet la résolution de la structure d'oligosaccharides de structure inconnue, en particulier d'oligosaccharides de plus de 2 monosaccharides, 3, 4, 5, 6 monosaccharides, voire plus de 10 monosaccharides, jusqu'à 20 monosaccharides ou plus.

Dans le cas de polysaccharides de très large structure, le séquençage peut se faire en plusieurs étapes, avec d'abord une fragmentation en oligosaccharides d'une vingtaine de monosaccharides, lesquels sont ensuite séquencés avec la méthode selon l'invention.

Par séquençage, on entend selon l'invention la résolution de la composition en monosaccharides, la position (régiochimie) et la configuration (stéréochimie) des liaisons glycosidiques, la nature et la position des modifications fonctionnelles, et sa structure branchée, dont en particulier l'identification de l'extrémité réductrice.

L'étape i. de fragmentation des oligosaccharides en disaccharides et monosaccharides est particulièrement importante puisqu'elle doit préserver la structure des constituants telle que présente dans l'oligosaccharide, c'est-à-dire conserver la nature même du sucre selon le positionnement et la stéréochimie des substituants hydroxyles sur le cycle, conserver la nature et la position des modifications fonctionnelles et permettre d'identifier la position (régiochimie) et la configuration (stéréochimie) des liaisons glycosidiques ainsi que la structure branchée et l'extrémité réductrice. Selon un mode préféré de réalisation de l'invention, la fragmentation est réalisée par spectrométrie de masse. En effet, les inventeurs ont pu montrer que la spectrométrie de masse permettait de conserver les informations structurales du précurseur lors de la fragmentation.

L'homme du métier saura déterminer les conditions de fragmentation de manière à préserver les informations structurales du précurseur, en particulier en utilisant par exemple les méthodes de fragmentation intégrées dans les spectromètres de masse commerciaux, telles que la CID (dissociation induite par collision ou « collision induced dissociation »), CAD (dissociation activée par collision ou « collision activated dissocation »), SID (dissociation induite de surface ou « surface induced dissociation »), ETD (dissociation par transfert d'électron ou « electron transfer dissociation »), ECD (dissociation par collision d'électron ou « electron collision dissociation »), et fragmentation induite par laser et préférentiellement la méthode CID

Les méthodes de spectrométrie de masse pour préparer des échantillons préalablement à une spectroscopie IR (étape ii.) sont bien connues, notamment décrites par Schindler, B. & al. (Phys. Chem. Chem. Phys. 2014, 16, 22131-22138).

L'homme du métier saura déterminer les conditions optimales de mise en oeuvre de cette étape de préparation d'échantillons, en particulier on générera le fragment d'intérêt (monosaccharide ou disaccharide constituant de l'oligosaccharide précurseur) par la méthode de fragmentation CID (dissociation induite par collision) en une ou plusieurs étapes de spectrométrie de masse MSⁿ, puis on l'isolera par spectrométrie de masse en préparation à la spectroscopie IR.

L'analyse par spectroscopie IR de sucres est connue de l'homme du métier, notamment décrite par Schindler, B. & al. (Phys. Chem. Chem. Phys. 2014, 16, 22131-22138).

Pour distinguer les différents sucres, leur structure et la position (régiochimie) et la configuration (stéréochimie) des liaisons glycosidiques on cherchera en particulier les informations relatives aux fréquences IR inférieures à 4000 cm⁻¹. Pour la nature et la position des modifications fonctionnelles, on cherchera en particulier les informations relatives aux fréquences IR inférieures à 4000 cm⁻¹. Selon un mode préféré de réalisation de l'invention, la spectroscopie IR (étape iii.) est réalisée à une longueur d'onde allant de 4000 à 2000 cm⁻¹.

L'homme du métier saura déterminer les conditions optimales de mise en oeuvre de cette spectroscopie, en fonction des méthodes de spectroscopie IR intégrées à un spectromètre de masse à sa disposition. Différentes méthodes de spectroscopie IR intégrées à un spectromètre de masse sont décrites dans la littérature et connues de l'homme du métier. On citera notamment la spectroscopie IRMPD (infrarouge par dissociation multiphotonique), la spectroscopie de double résonance UV/IR, la spectroscopie par attachement d'hydrogène ou d'hélium. De manière préférentielle, la spectroscopie sera faite par la méthode IRMPD implémentée en piège d'ions, permettant de produire les fragments par spectrométrie de masse et de les analyser avec un seul montage instrumental intégré, comme décrit par Schindler, B. & al. (Phys. Chem. Chem. Phys. 2014, 16, 22131-22138).

L'identification de la structure de chaque disaccharide et monosaccharide est faite par comparaison des spectres IR obtenus à un ensemble de spectres IR de disaccharides et monosaccharides de référence préalablement réalisés.

L'identification se fait par comparaison de l'empreinte spectroscopique obtenue avec les empreintes spectroscopiques de référence, en particulier par comparaison des positions et des intensités des bandes de vibration dans la gamme spectrale 2000-4000 cm⁻¹, par toute méthode de comparaison visuelle effectuée par l'opérateur ou toute méthode informatisée connue de l'homme du métier.

Ces empreintes spectroscopiques de référence (spectres de référence) sont obtenues par la même méthode spectroscopique que celle mise en oeuvre pour le procédé selon l'invention (étapes ii de spectrométrie de masse préparatrice d'échantillons et étape iii. de spectroscopie IR). Elles sont obtenues pour des composés incluant les monosaccharides (mélange anomérique), les formes anomériques pures des monosaccharides (soit naturelles soit modifiées chimiquement, par exemple par méthylation du groupement hydroxyle réducteur), les formes deshydroxylées des monosaccharides, les disaccharides et les formes deshydroxylées des disaccharides. Pour chaque type de composé de référence, on analysera les différentes formes fonctionnalisées connues et leurs isomères de position.

De manière avantageuse, ces spectres de référence sont assemblés et conservés dans une base de données ou librairie. Cette base de données peut être une base de données « physique » où les spectres imprimés sur support approprié, en particulier papier, sont classés selon le type de sucre et conservés en un même lieu, accessibles pour les comparaisons de spectres. Selon un mode préféré de réalisation de l'invention, la base de données est une base de données dématérialisée où l'ensemble des informations sur les mono- et disaccharides, leurs structures, les spectres associés, les bandes caractéristiques et leurs intensités relatives sont conservées sous une forme numérique, conservée sur support informatique, ordinateur, serveur, serveur dématérialisé (« cloud »). Dans ce cas, l'accès à la base de données se fait par tout moyen de communication approprié. La comparaison des spectres obtenus pour les mono- et dissacharides issus de la fragmentation de l'oligosaccharide à analyser se fait alors par les mêmes moyens « informatiques », notamment par des méthodes bien connues de comparaison de données, qu'il s'agisse d'images (spectres) ou de valeurs obtenues à partir de ces spectres.

Dans l'éventualité où la base de données ne comprendrait pas un spectre de référence associé à un mono- ou disaccharide particulier issu de la fragmentation d'un oligosaccharide de structure inconnue, l'homme du métier sera à même de compléter la base de données par des monosaccharides et disaccharides de référence pertinents, en particulier en estimant les structures possibles sur la base des informations obtenues par spectrométrie de masse et par spectroscopie IR.

La connaissance des informations relatives à chaque di- et monosaccharide constitutif de l'oligosaccharide analysé, en particulier la connaissance de la position et la configuration de leurs liaisons osidiques, permet de déterminer la séquence de l'oligosaccharide de départ en combinant les différentes structures. Ces méthodes combinatoires sont décrites ci-après.

En particulier, la combinaison des informations obtenues avec les monosaccharides et les disaccharides est nécessaire et suffisante pour pouvoir résoudre la structure de l'oligosaccharide.

La composition en monosaccharides et la configuration (stéréochimie) des liaisons glycosidiques, la nature et la position des modifications fonctionnelles, et la structure branchée, dont en particulier l'identification de l'extrémité réductrice de l'oligosaccharide analysé sont identifiés par correspondance des empreintes spectroscopiques des monosaccharides constituants avec la librairie de référence de la manière suivante:
- l'empreinte spectroscopique mesurée pour chacun des monosaccharides constituants est comparée à la section adéquate de la librairie de référence, c'est à dire la section comprenant les isomères de référence correspondant à la masse du fragment mesurée dans l'étape 1.
- une correspondance pour les fragments deshydroxylés est obtenue dans la librairie de référence de monosaccharides deshydroxylés ce qui permet d'identifier la nature de chacun des monosaccharides constituants et la position de leurs modifications fonctionelles le cas échéant. On obtient ainsi la composition en monosaccharides de l'oligosaccharide parent et la position des modifications fonctionnelles.
- une correspondance pour les fragments complets est obtenue dans la librairie de référence de monosaccharides standards et de leurs formes anomériques pures ce qui permet d'identifier la nature de chacun des monosaccharides constituants et la position de leurs modifications fonctionelles le cas échéant. On obtient ainsi la composition en monosaccharides de l'oligosaccharide parent et la position des modifications fonctionnelles (information potentiellement redondante avec l'analyse des fragments deshydroxylés).
- les fragments complets ayant une correspondance dans la librairie de monosaccharides (mélange anomérique) sont identifiés comme des extrémités réductrices.
- les fragments complets ayant une correspondance dans la librairie de monosaccharides de forme anomérique pure sont identifiés comme des extrémités non réductrices et leur configuration anomérique est identifiée.

La composition en monosaccharides, la position (régiochimie) et la configuration (stéréochimie) des liaisons glycosidiques, la nature et la position des modifications fonctionnelles de l'oligosaccharide, et sa structure branchée, dont en particulier l'identification de l'extrémité réductrice de l'oligosaccharide parent sont identifiés par correspondance des empreintes spectroscopiques des disaccharides constituants avec la librairie de référence de la manière suivante:
- l'empreinte spectroscopique mesurée pour chacun des disaccharides constituants est comparée à la section adéquate de la librairie de référence, c'est-à-dire la section comprenant les isomères de référence correspondant à la masse du fragment.
- connaissant la composition en monosaccharides, la position des modifications fonctionnelles et la configuration anomérique des liaisons, la correspondance des disaccharides permet d'identifier la position de la liaison glycosidique.
- alternativement, l'analyse des disaccharides par correspondance avec la librairie de référence permet d'identifier leur composition en monosaccharide, la position des modifications fonctionnelles, la configuration anomérique et la position de la liaison glycosidique.

L'invention concerne aussi un appareil de séquençage de oligosaccharides comprenant un dispositif de spectrométrie de masse, une source de rayonnement électromagnétique, une base de données et des moyens de traitement, caractérisé en ce qu'il comprend un processeur pour commander les étapes de
i. fragmentation des oligosaccharides en disaccharides et monosaccharides en préservant la structure moléculaire des constituants telle que présente dans l'oligosaccharide à séquencer
ii. séparation de chaque disaccharide et monosaccharide obtenus précédemment par spectrométrie de masse avec le dispositif de spectrométrie de masse,
iii. analyse par spectroscopie vibrationnelle IR (infrarouge) de chaque disaccharide et monosaccharide séparés précédemment avec la source de rayonnement électromagnétique,
iv. identification de la structure de chaque disaccharides et monosaccharides par comparaison des spectres IR obtenus à un ensemble de spectres IR de disaccharides et monosaccharides de référence contenus dans la base de données, et
v. définition de la séquence de l'oligosaccharide par combinaison des structures identifiées pour chaque disaccharide et monosaccharide par les moyens de traitement.

En particulier, la source de rayonnement électromagnétique est une source L.A.S.E.R. La source de rayonnement électromagnétique est avantageusement intégrée au dispositif de spectrométrie de masse.

### DESCRIPTION DES FIGURES

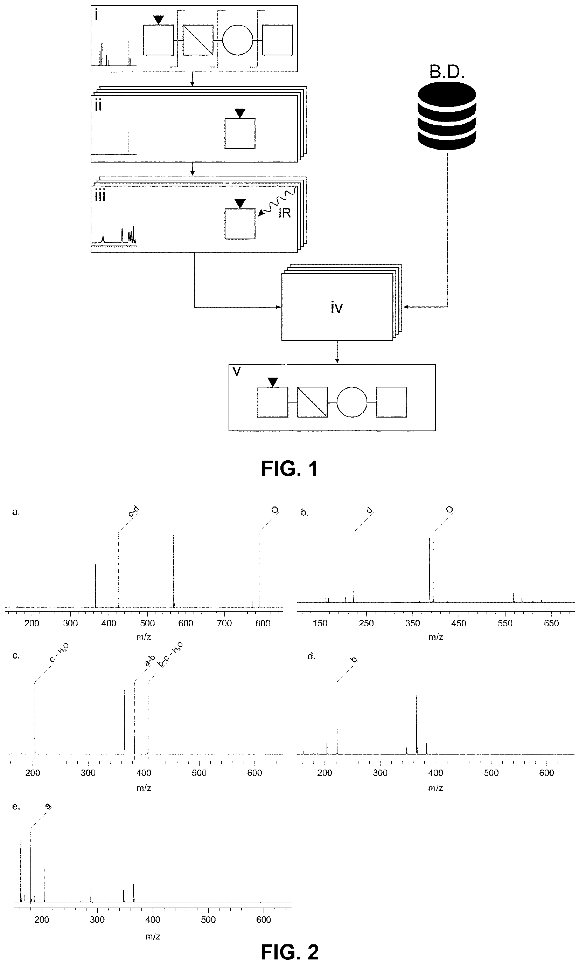

La **figure 1** représente de manière schématique la mise en oeuvre du procédé selon l'invention : (i) fragmentation de l'oligosaccharide par spectrométrie de masse, (ii) séparation de chacun des fragments en préparation à l'analyse par spectroscopie IR, (iii) analyse par spectroscopie IR, (iv) identification de la structure de chaque constituant, monosaccharide et disaccharide par comparaison avec les spectres IR de référence contenus dans la base de données BD, (v) définition de la séquence par la méthode combinatoire décrite dans l'invention.
Les figures 2, 3 et 4 se rapportent à la mise en oeuvre de la méthode sur l'exemple du tétrasaccharide GlcNβ(1⊙4)GlcNAcβ(1⊙4)GlcNAcβ(1⊙4)GlcNAc
La **figure 2** représente la production de l'ensemble des fragments monosaccharides (notés a, b, c, d) et disaccharides (notés a-b, b-c, c-d) constituants du tétrasaccharide (noté O) par plusieurs étapes de spectrométrie de masse MSⁿ.
La **figure 3** représente l'analyse de chacun des fragments monosaccharides par spectroscopie IRMPD (cadres du haut) et leur comparaison aux spectres de référence avec lesquels les spectres des fragments ont été identifiés (cadres du bas).
La **figure 4** représente l'analyse de chacun des fragments disaccharides par spectroscopie IRMPD (cadres du haut) et leur comparaison aux spectres de référence avec lesquels les spectres des fragments ont été identifiés (cadres du bas).

### EXEMPLES

### 1. Matériel et Méthodes

### Matériel

Le matériel employé pour la mise en oeuvre de la méthode de l'invention comprend Un spectromètre de masse commercial de type piège à ions équipé d'une source d'ions électrospray de modèle LCQ Thermofinnigan. Cet appareil est modifié pour permettre l'injection d'un faisceau L.A.S.E.R. infrarouge généré par un système OPO/OPA accordable de la marque LaserVision pompé par Yag à une cadence de 10 Hz. Il est notamment décrit dans Schindler, B. & al. (Phys. Chem. Chem. Phys. 2014, 16, 22131-22138)

### Fragmentation

La fragmentation des échantillons se fait par la méthode CID, en plusieurs étapes de fragmentations successives le cas échéant.

### Échantillonnage et Spectroscopie IR

La méthode de préparation des échantillons par MSⁿ et spectroscopie IRMPD est celle décrite dans Schindler, B. & al. (Phys. Chem. Chem. Phys. 2014, 16, 22131-22138).

### 2. Analyse d'un tétrasaccharide

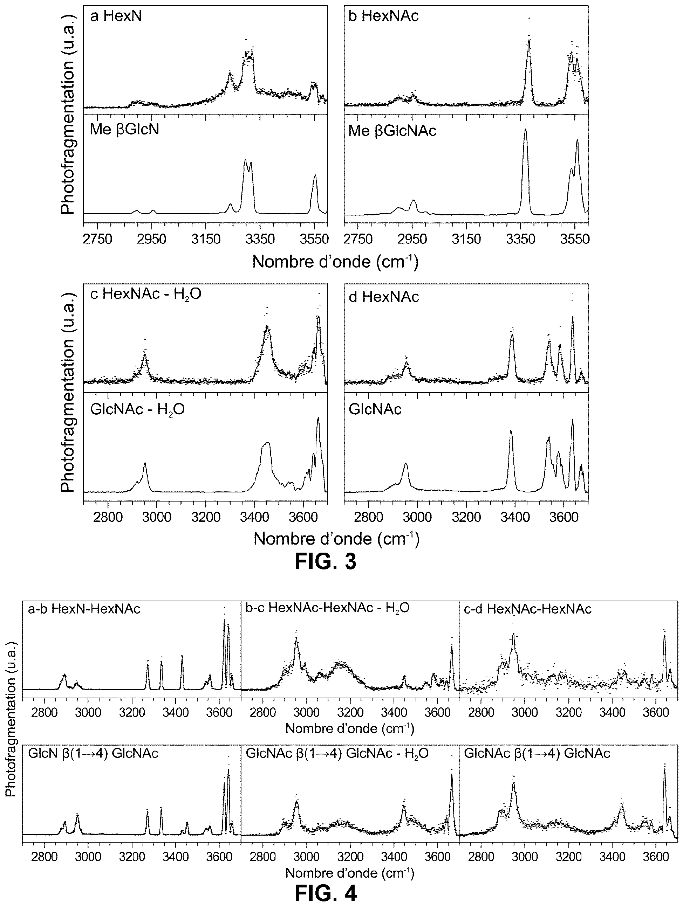

Les figures 2, 3 et 4 se rapportent à la mise en oeuvre de la méthode précédemment décrite sur l'exemple du tétrasaccharide

GlcNβ(1⊙4)GlcNAcβ(1⊙4)GlcNAcβ(1⊙4)GlcNAc.

L'ensemble des informations structurales obtenues par comparaison des spectres des fragments et des spectres de référence sont listées dans le Tableau 1

**Tableau 1. Identification de la structure de chaque disaccharide et monosaccharide par comparaison des spectres IR obtenus à un ensemble de spectres IR de disaccharides et monosaccharides de référence.**

| identification de la structure de monosaccharides | | | | |
|---|---|---|---|---|
| Fragment | MSⁿ | m/z | Type générique | identification |
| a | MS⁴ | 180 | HexN | β GlcN |
| b | MS⁴ | 222 | HexNac | β GlcNAc |
| c | MS³ | 204 | HexNac-H₂O | GlcNAc-H₂O |
| d | MS² | 222 | HexNac | GlcNAc |

| identification de la structure de disaccharides | | | | |
|---|---|---|---|---|
| Fragment | MSⁿ | m/z | Type générique | identification |
| a-b | MS³ | 383 | HexN-HexNAc | GlcN β(1→4) GlcNAc |
| b-c | MS³ | 407 | HexNAc-HexNAc-H₂O | GlcNAc β(1→4) GlcNAc-H₂O |
| c-d | MS² | 425 | HexNAc-HexNAc | GlcNAc β(1→4) GlcNAc |

La séquence de l'oligosaccharide est alors obtenue par combinaison des informations structurales :

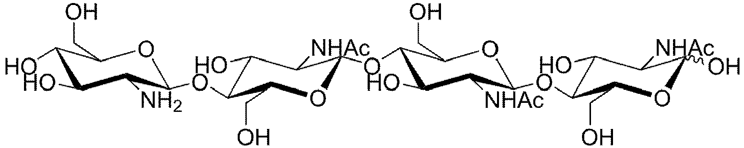

La structure obtenue est bien celle du tétrasaccharide GlcNβ(1⊙4)GlcNAcβ(1⊙4)GlcNAcβ(1⊙4)GlcNAc.

### REFERENCES

- Both, P. & al., Discrimination of epimeric glycans and glycopeptides using IM-MS and its potential for carbohydrate sequencing. Nat. Chem. 2013, 6, 65-74.
- Gaye, M. M. & al., Multidimensional Analysis of 16 Glucose Isomers by Ion Mobility Spectrometry. Anal. Chem. 2016, 88, 2335-2344.
- Nagy, G.; Pohl, N. L. B., Monosaccharide identification as a first step toward de novo carbohydrate sequencing: Mass spectrometry strategy for the identification and differentiation of diastereomeric and enantiomeric pentose isomers. Anal. Chem. 2015, 87, 677-685.
- Nagy, G.; Pohl, N. L. B., Complete Hexose Isomer Identification with Mass Spectrometry. J. Amer. Soc. Mass Spectrom. 2015, 26, 677-685.
- Schindler, B. & al., Distinguishing isobaric phosphated and sulfated carbohydrates by coupling of mass spectrometry with gas phase vibrational spectroscopy. Phys. Chem. Chem. Phys. 2014, 16, 22131-22138
- Stefan, S. & al., Differentiation of Closely Related Isomers: Application of Data Mining Techniques in Conjunction with Variable Wavelength Infrared Multiple Photon Dissociation Mass Spectrometry for Identification of Glucose-Containing Disaccharide Ions. Anal. Chem. 2011, 83, 8468-8476

## Revendications

1. Procédé de séquençage de oligosaccharides **caractérisé en ce qu'**il comprend les étapes de
i. fragmentation des oligosaccharides en disaccharides et monosaccharides en préservant la structure moléculaire des constituants telle que présente dans l'oligosaccharide à séquencer
ii. séparation de chaque disaccharide et monosaccharide obtenus précédemment par spectrométrie de masse,
iii. analyse par spectroscopie vibrationnelle infrarouge ou ≪ IR ≫ de chaque disaccharide et monosaccharide séparés précédemment,
iv. identification de la structure de chaque disaccharide et monosaccharide par comparaison des spectres IR obtenus à un ensemble de spectres IR de disaccharides et monosaccharides de référence, et
v. définition de la séquence de l'oligosaccharide par combinaison des structures identifiées pour chaque disaccharide et monosaccharide.

2. Procédé selon la revendication 1, **caractérisé en ce que** la fragmentation des oligosaccharides en disaccharides et monosaccharides dans l'étape i. est faite par spectrométrie de masse.

3. Procédé selon l'une des revendications 1 ou 2, **caractérisé en ce que** la fragmentation par spectrométrie de masse est faite par dissociation induite par collision ou « CID », dissociation activée par collision ou « CAD », dissociation induite de surface ou « SID », dissociation par transfert d'électron ou « ETD », dissociation par collision d'électron ou « ECD » et fragmentation induite par laser.

4. Procédé selon l'une des revendications 1 à 3, caractérisé en que ce que la spectroscopie IR est réalisée à une longueur d'onde allant de 4000 cm⁻¹ à 2000 cm⁻¹.

5. Procédé selon l'une des revendications 1 à 4, **caractérisée en ce que** la spectroscopie IR est faite par la méthode infrarouge par dissociation multiphotonique ou ≪ IRMPD ≫ implémentée en piège d'ions.

6. Appareil de séquençage d'oligosaccharides comprenant un dispositif de spectrométrie de masse, une source de rayonnement électromagnétique, une base de données et des moyens de traitement, **caractérisé en ce qu'**il comprend un processeur pour commander les étapes de
i. fragmentation des oligosaccharides en disaccharides et monosaccharides en préservant la structure moléculaire des constituants telle que présente dans l'oligosaccharide à séquencer
ii. séparation de chaque disaccharide et monosaccharide obtenus précédemment par spectrométrie de masse avec le dispositif de spectrométrie de masse,
iii. analyse par spectroscopie vibrationnelle infrarouge ou ≪ IR ≫ de chaque disaccharide et monosaccharide séparés précédemment avec la source de rayonnement électromagnétique,
iv. identification de la structure de chaque disaccharide et monosaccharide par comparaison des spectres IR obtenus à un ensemble de spectres IR de disaccharides et monosaccharides de référence contenus dans la base de données, et
v. définition de la séquence de l'oligosaccharide par combinaison des structures identifiées pour chaque disaccharide et monosaccharide par les moyens de traitement.

7. Appareil selon la revendication 6, **caractérisé en ce que** la source de rayonnement électromagnétique est une source L.A.S.E.R.

8. Appareil selon l'une des revendications 6 ou 7, **caractérisé en ce que** la source de rayonnement électromagnétique est intégrée au dispositif de spectrométrie de masse.

## Patentansprüche

1. Verfahren zur Sequenzierung von Oligosacchariden, **dadurch gekennzeichnet, dass** es die Schritte umfasst
i. Fragmentieren der Oligosaccharide in Disaccharide und Monosaccharide bei Beibehaltung der molekularen Struktur der Bestandteile so wie in dem zu sequenzierenden Oligosaccharid vorhanden
ii. Trennen jedes zuvor erhaltenen Disaccharids und Monosaccharids durch Massenspektrometrie,
iii. Analysieren durch Infrarot- oder "IR"-Vibrationsspektroskopie jedes zuvor getrennten Disaccharids und Monosaccharids,
iv. Identifizieren der Struktur jedes Disaccharids und Monosaccharids durch Vergleich der erhaltenen IR-Spektren mit einer Einheit von IR-Spektren von Referenz-Disacchariden und - Monosacchariden, und
v. Definieren der Sequenz des Oligosaccharids durch Kombination der für jedes Disaccharid und Monosaccharid identifizierten Strukturen.

2. Verfahren nach Anspruch 1, **dadurch gekennzeichnet, dass** das Fragmentieren der Oligosaccharide in Disaccharide und Monosaccharide in Schritt i. durch Massenspektrometrie erfolgt.

3. Verfahren nach einem der Ansprüche 1 oder 2, **dadurch gekennzeichnet, dass** das Fragmentieren durch Massenspektrometrie durch kollisionsinduzierte Dissoziation oder "CID", kollisionsaktivierte Dissoziation oder "CAD", oberflächeninduzierte Dissoziation oder "SID", Elektronentransfer-Dissoziation oder "ETD", Elektronenkollisions-Dissoziation oder "ECD" und laserinduziertes Fragmentieren erfolgt.

4. Verfahren nach einem der Ansprüche 1 bis 3, **dadurch gekennzeichnet, dass** die IR-Spektroskopie bei einer Wellenlänge von 4000 cm⁻¹ bis 2000 cm⁻¹ durchgeführt wird

5. Verfahren nach einem der Ansprüche 1 bis 4, **dadurch gekennzeichnet, dass** die IR-Spektroskopie durch die in Ionenfalle implementierte Infrarot-Multiphotonendissoziations- oder "IRMPD"-Methode durchgeführt wird.

6. Apparat zur Sequenzierung von Oligosacchariden, umfassend eine Massenspektrometrievorrichtung, eine elektromagnetische Strahlungsquelle, eine Datenbank und Verarbeitungsmittel, **dadurch gekennzeichnet, dass** er einen Prozessor zum Steuern der Schritte umfasst
i. Fragmentieren der Oligosaccharide in Disaccharide und Monosaccharide bei Beibehaltung der molekularen Struktur der Bestandteile so wie in dem zu sequenzierenden Oligosaccharid vorhanden
ii. Trennen jedes zuvor erhaltenen Disaccharids und Monosaccharids durch Massenspektrometrie mit der Massenspektrometrievorrichtung,
iii. Analysieren durch Infrarot- oder "IR"-Vibrationsspektroskopie jedes zuvor getrennten Disaccharids und Monosaccharids mit der elektromagnetischen Strahlungsquelle,
iv. Identifizieren der Struktur jedes Disaccharids und Monosaccharids durch Vergleich der erhaltenen IR-Spektren mit einer Einheit von IR-Spektren von Referenz-Disacchariden und - Monosacchariden, die in der Datenbank enthalten sind, und
v. Definieren der Sequenz des Oligosaccharids durch Kombination der für jedes Disaccharid und Monosaccharid identifizierten Strukturen durch die Verarbeitungsmittel.

7. Apparat nach Anspruch 6, **dadurch gekennzeichnet, dass** die elektromagnetische Strahlungsquelle eine LASER-Quelle ist.

8. Apparat nach einem der Ansprüche 6 oder 7, **dadurch gekennzeichnet, dass** die elektromagnetische Strahlungsquelle in die Massenspektrometrievorrichtung integriert ist.

## Claims

1. Process for sequencing oligosaccharides, **characterized in that** it comprises the steps of
i. fragmentation of the oligosaccharides into disaccharides and monosaccharides while preserving the molecular structure of the constituents as present in the oligosaccharide to be sequenced
ii. separation of each previously obtained disaccharide and monosaccharide by mass spectrometry,
iii. analysis by infrared or "IR" vibrational spectroscopy of each previously separated disaccharide and monosaccharide,
iv. identification of the structure of each disaccharide and monosaccharide by comparison of the obtained IR spectra with a set of reference disaccharide and monosaccharide IR spectra, and
v. definition of the oligosaccharide sequence by combination of the structures identified for each disaccharide and monosaccharide.

2. Process according to claim 1, **characterized in that** the fragmentation of the oligosaccharides into disaccharides and monosaccharides in step i. is done by mass spectrometry.

3. Process according to one of claims 1 or 2, **characterized in that** the fragmentation by mass spectrometry is done by collision-induced dissociation (CID), collision-activated dissociation (CAD), surface-induced dissociation (SID), electron-transfer dissociation (ETD), electron-collision dissociation (ECD) and laser induced fragmentation.

4. Process according to one of claims 1 to 3, **characterized in that** the IR spectroscopy is performed at a wavelength ranging from 4000 cm⁻¹ to 2000 cm⁻¹.

5. Process according to one of claims 1 to 4, **characterized in that** the IR spectroscopy is done by the infrared multiphoton dissociation or "IRMPD" method implemented in an ion trap.

6. Apparatus for sequencing oligosaccharides comprising a mass spectrometry device, an electromagnetic radiation source, a database and processing means, **characterized in that** it comprises a processor for controlling the steps of
i. fragmentation of the oligosaccharides into disaccharides and monosaccharides while preserving the molecular structure of the constituents as present in the oligosaccharide to be sequenced
ii. separation of each previously obtained disaccharide and monosaccharide by mass spectrometry with the mass spectrometry device,
iii. analysis by infrared or "IR" vibrational spectroscopy of each previously separated disaccharide and monosaccharide with the electromagnetic radiation source,
iv. identification of the structure of each disaccharide and monosaccharide by comparison of the obtained IR spectra with a set of reference disaccharide and monosaccharide IR spectra contained in the database, and
v. definition of the oligosaccharide sequence by combination of the structures identified for each disaccharide and monosaccharide by the processing means.

7. Apparatus according to claim 6, **characterized in that** the electromagnetic radiation source is a L.A.S.E.R. source.

8. Apparatus according to one of claims 6 or 7, **characterized in that** the electromagnetic radiation source is integrated into the mass spectrometry device.
